# EUROPEAN PATENT APPLICATION

(11) **EP 3 646 882 A1**
(43) Date of publication of application: **06.05.2020**
(21) Application number: 18204287.9
(22) Date of filing: 05.11.2018
(51) Int. Cl.: A61K 39/00, A61K 39/39, A61P 37/04

(54) **PHARMACEUTICAL COMPOSITIONS, VACCINES AND THEIR USES IN THE PREVENTION OR TREATMENT OF A PERSISTENT INFECTION OR OF CANCER**

(71) Applicant: Universitätsmedizin der Johannes Gutenberg-Universität Mainz, 55131 Mainz (DE)
(72) Inventor: RADSAK, Markus, 55126 Mainz (DE); SOHL, Julian, 65779 Kelkheim (DE); STASSEN, Michael, 55291 Saulheim (DE); HARTMANN, Ann-Kathrin, 55299 Nackenheim (DE)
(74) Representative: Patentanwälte Dr. Keller, Schwertfeger Partnerschaft mbB

(57) **Abstract**

The present invention relates to pharmaceutical compositions, comprising at least one oxidative stress substance selected from the group consisting of dithranol (anthralin, cignolin), or other anthrones or hydroxyanthracenes, at least one Toll-like receptor 7 (TLR7) ligand, and at least one peptide antigen. The invention further relates pharmaceutical combination preparations comprising these components and the use of such pharmaceutical compositions or combination preparations for use in the prevention or treatment of a persistent viral, bacterial or fungal infection or of cancer. The pharmaceutical compositions or combination preparations of the invention are in particular useful for topical application on the skin of a human or animal body.

## Description

The present invention relates to pharmaceutical compositions, comprising at least one oxidative stress substance selected from the group consisting of dithranol (anthralin, cignolin), or other anthrones or hydroxyanthracenes, at least one Toll-like receptor 7 (TLR7) ligand, and at least one peptide antigen. The invention further relates pharmaceutical combination preparations comprising these components and the use of such pharmaceutical compositions or combination preparations for use in the prevention or treatment of a persistent viral, bacterial or fungal infection or of cancer. The pharmaceutical compositions or combination preparations of the invention are in particular useful for topical application on the skin of a human or animal body.

An activation of the innate immune system is a prerequisite to initiate adaptive immune responses. One of the major pathways eliciting these responses is the recognition of foreign bodies through Toll-like receptors (TLR), which results in the activation of antigen presenting cells (APC) and the production of a variety of pro-inflammatory mediators (Takeda K et al., Toll-like receptors, Annu Rev Immunol (2003), Vol. 21:335-376).

TLRs recognize a variety of exogenous and endogenous ligands, and the binding of natural and synthetic TLR ligands to TLRs induces the production of multiple cytokines, resulting in an enhanced innate and adaptive immunity (Vasilakos JP, Tomai MA. The use of Toll-like receptor 7/8 agonists as vaccine adjuvants. Expert Rev Vaccines. 2013; 12:809-19; Aranda F, Vacchelli E, Obrist F, Eggermont A, Galon J, Sautes-Fridman C, Cremer I, Henrik Ter Meulen J, Zitvogel L, Kroemer G, Galluzzi L. Trial Watch: Toll-like receptor agonists in oncological indications. Oncoimmunology. 2014; 3:e29179; Dowling JK, Mansell A. Toll-like receptors: the swiss army knife of immunity and vaccine development. Clin Transl Immunology. 2016; 5:e85). Small compounds that are designed to bind TLRs are potential drugs for vaccines and adjuvants in infectious disease and cancer. TLR7 and TLR8, which are expressed on endosomes, have structural similarity and recognize microbial single-stranded RNA (ssRNA). Imiquimod (R837) and resiquimod (R848), which are imidazoquinoline compounds of synthesized ssRNAs, bind TLR7 alone and both TLR7 and TLR8, respectively (Dockrell DH, Kinghorn GR. Imiquimod and resiquimod as novel immunomodulators. J Antimicrob Chemother. 2001; 48:751-5; Schon MP, Schon M. TLR7 and TLR8 as targets in cancer therapy. Oncogene. 2008; 27:190-9). Resiquimod does not exert mouse TLR8- mediated activation however. TLR7/8-mediated signaling induces the production of NF-kB-mediated proinflammatory cytokines and chemokines, and type I interferon (IFN) by APCs, resulting in the augmentation of effector T-cells (Vasilakos JP, Tomai MA. The use of Toll-like receptor 7/8 agonists as vaccine adjuvants. Expert Rev Vaccines. 2013; 12:809-19; Smits EL, Ponsaerts P, Berneman ZN, Van Tendeloo VF. The use of TLR7 and TLR8 ligands for the enhancement of cancer immunotherapy. Oncologist. 2008; 13:859-75; Meyer T, Surber C, French LE, Stockfleth E. Resiquimod, a topical drug for viral skin lesions and skin cancer. Expert Opin Investig Drugs. 2013; 22:149-59).

Some imidazoquinolines have been applied in a clinical setting as a locally administrated drug for the treatment of non-melanoma skin cancers and viral skin lesions to avoid TLR tolerance and adverse proinflammatory and proapoptotic effects (Hayashi T, Gray CS, Chan M, Tawatao RI, Ronacher L, McGargill MA, Datta SK, Carson DA, Corr M. Prevention of autoimmune disease by induction of tolerance to Toll-like receptor 7. Proc Natl Acad Sci U S A. 2009; 106:2764-9; Micali G, Lacarrubba F, Nasca MR, Schwartz RA. Topical pharmacotherapy for skin cancer: part I. Pharmacology. J Am Acad Dermatol. 2014; 70:965.e1-12; quiz 77-8; Mark KE, Spruance S, Kinghorn GR, Sacks SL, Slade HB, Meng TC, Selke S, Magaret A, Wald A. Three phase III randomized controlled trials of topical resiquimod 0.01-percent gel to reduce anogenital herpes recurrences. Antimicrob Agents Chemother. 2014; 58:5016-23; Rook AH, Gelfand JM, Wysocka M, Troxel AB, Benoit B, Surber C, Elenitsas R, Buchanan MA, Leahy DS, Watanabe R, Kirsch IR, Kim EJ, Clark RA. Topical resiquimod can induce disease regression and enhance T-cell effector functions in cutaneous T-cell lymphoma. Blood. 2015; 126:1452-61).

Recently, systemic or intratumoral administration of TLR7 agonists, such as resiquimod and its related conjugate, has shown to induce efficient tumor regression by inhibiting TAM- or MDSC-mediated immune suppression in murine tumor models (Spinetti T, Spagnuolo L, Mottas I, Secondini C, Treinies M, Ruegg C, Hotz C, Bourquin C. TLR7-based cancer immunotherapy decreases intratumoral myeloid-derived suppressor cells and blocks their immunosuppressive function. Oncoimmunology. 2016; 5:e1230578; Sato-Kaneko F, Yao S, Ahmadi A, Zhang SS, Hosoya T, Kaneda MM, Varner JA, Pu M, Messer KS, Guiducci C, Coffman RL, Kitaura K, Matsutani T, et al. Combination immunotherapy with TLR agonists and checkpoint inhibitors suppresses head and neck cancer. JCI Insight. 2017; 2:e93397). In PD-1 blockade therapy-resistant and immunosuppressive tumors, such as HNC, combined treatment with a TLR7 agonist and PD-1 blockade might be an effective cancer immunotherapy. In this study, the effects of monotherapy of systemic low-dose resiquimod and combined therapy with anti-PD-L1 treatment in two PD-L1 blockade-resistant tumor models were examined.

Cytotoxic T-cells have been a direct or indirect clinical target in order to eliminate intracellular pathogens or tumor cells. Recent immunization strategies attempt to activate T-cells, in particular cytotoxic T-cells, but the methods lack the ability to generate efficient T-cell responses having a long-term effect. Usually, such responses are mediated by memory cells of the immune system in order to initiate a sustainable immune response.

US 9,017,654 B2 describes a pharmaceutical preparation which comprises at least one TLR7 ligand and peptide which qualifies as MHCI ligand or MHCII ligand having the capability of being presented by at least one of a MHC class I molecule and a MHC class II molecule. The pharmaceutical preparation is utilized for diseases that are induced by viruses, bacteria or fungi. However, this preparation is not able to elicit efficient therapeutic immune responses, the underlying problem of which is that the induced immune responses cannot trigger sustainable memory immune responses, in particular therapeutically effective memory T-cell responses.

An alternative approach suggests a systemic resiquimod administration immunotherapy and a combined treatment with PD-L1 blockade in two PHTL1 blockade-resistant tumor models that exhibit different profiles of TILs (Nishii N et al., onco-target (2018), Vol. 9: 13301-1312). Again, this approach will not trigger memory T-cell responses resulting in the recognition and destruction of virus-induced cells or tumor cells.

An alternative approach is disclosed in US 2017/0100477 A1 which describes anthralin as an adjuvant for influenza vaccination in combination with an effective amount of an antigen or vaccine. Imiquimod combined with a vaccine was used for an adjuvant effect to improve survival of mice when compared to the vaccine alone.

It is therefore the object of the present invention to provide pharmaceutical compositions and combination preparations with the ability to induce therapeutically effective memory T-cell responses in order to prevent or treat a persistent viral, bacterial or fungal infection or of a neoplastic disease such as cancer.

This object is solved by a pharmaceutical composition or a pharmaceutical combination preparation that comprises at least the following components: (a) at least one oxidative stress substance selected from the group consisting of dithranol (anthralin, cignolin), or other anthrones or hydroxyanthracenes, (b) at least one Toll-like receptor 7 (TLR7) ligand, and (c) at least one peptide antigen.

Surprisingly, it has been found that a simultaneous or successive administration of an oxidative stress substance such as dithranol will intensify a synergistic activation of cytotoxic T-cells when administered in combination with at least one TLR7 ligand and a peptide antigen that contains an antigen epitope recognized by T effector cells. The synergistic combination triggers an improved systemic T-cell imparted immune response and subsequently activates memory T-cell responses which makes the recognition and destruction of pathogens and tumor cells more efficient. As such, the inventive preparation is more efficient in comparison to the combination of TLR7 ligand and peptide antigen alone without application of an oxidative stress substance such as dithranol. The dithranol as used in the present invention is a compound that is a hydroxyanthrone, anthracene derivative, preferably 1,8-dihydroxy-9-anthrone.

The synergistic effects of the three compounds and their application as medical drug makes the pharmaceutical compositions and preparations suitable for a treatment of patients that suffer from a progressive tumor disease or the system pathogen infections, in particular virus infections. The pharmaceutical compositions and preparations of the invention are preferably suitable for topical administration on the skin of a patient (human or animal) mediated by a transcutaneous vaccination.

In a preferred embodiment, the TLR7 ligand of the inventive composition or combination preparation is imiquimod (R837), loxoribine and/or resiquimod (R848). The invention also encompasses analogues or modified forms of imiquimod, loxoribine and/or resiquimod having the same biological or pharmaceutical activity, resulting in the same or similar therapeutic effect. The TLR7 ligand of the inventive composition can be of any origin, i.e. natural and/or synthetic origin, as long as they have the capability of being recognized by the TLR7 receptor and being capable of triggering one or more signals to promote an immune response. As such, also other TLR7 ligands such as Pam3Cys, poly-(I:C) or CpG-DNA are suitable compounds for use in the compositions and combination preparations according to the present invention.

In a preferred embodiment, the at least one peptide antigen of the pharmaceutical compositions of the invention is selected from the group consisting of (i) major histocompatibility complex class I (MHCI) ligands, (ii) major histocompatibility complex class II (MHCII) ligands, or (iii) peptides that comprise one or more of said MHCI and/or MHCII ligands. The peptide antigen has the ability to be recognized by cytotoxic T-cells and is preferably a peptide of viral, fungal or bacterial origin. Alternatively, tumor-specific peptides or tumor-associated peptides that are presented by MHC class I molecules or by MHC class II molecules are encompassed by the present invention.

The term "peptide antigen" according to the present invention not only refers to peptides, but also to polypeptides, and proteins.

Suitable peptide antigens can be any synthetic peptides or isolated naturally occurring peptides comprising or consisting of one of the following amino acid sequences: SEQ ID NO: 1 (PLDGEYFTL), SEQ ID NO: 2 (YMNGTMSQV),SEQ ID NO: 3 (AAGIGILTV), SEQ ID NO: 4 (VLRENTSPK), SEQ ID NO: 5 (SPSSNRIRNT), SEQ ID NO: 6 (RLVTLKDIV), SEQ ID NO: 7 (AVFDRKSDAK), SEQ ID NO: 8 (GLSPTVWLSV), SEQ ID NO: 9 (ILKEPVHGV), SEQ ID NO: 10 (KIRLRPGGK), SEQ ID NO: 11 (TPGPGVRYPL), SEQ ID NO: 12 (ILGFVFTLTV), SEQ ID NO: 13 (VLTDGNPPEV), SEQ ID NO: 14 (TQHFVQENYLEY), SEQ ID NO: 15 (WRRAPAPGAKAMAPG), SEQ ID NO: 16 (FRKQNPDIVIQYMDDLYVG), SEQ ID NO: 17 (RIHIGPGRAFYTTKNIIGTI), SEQ ID NO: 18 (PGPLRESIVCYFMVFLQTHI), SEQ ID NO: 19 (PYYTGEHAKAIGN), ), SEQ ID NO: 20, (IAFNSGMEPGVVAEKV), SEQ ID NO: 21 (KQEELERDLRKTKKKI), SEQ ID NO: 22 (GRDIKVQFQSGGNNSPAV), SEQ ID NO: 23 (SIINFEKL), SEQ ID NO: 24 (SIIQFEHL), SEQ ID NO: 25 (SGPSNTPPEI) and modified forms thereof in which one or more amino acids in said amino acid sequences are linked to a chemical group, preferably NH₂ or CH₃.

In a preferred embodiment, peptide antigens consist of anyone of the amino acid sequences of SEQ ID NO:1 to SEQ ID NO:25. The invention also covers modified forms of said peptide antigens in which one or more amino acids in these amino acid sequences are linked to one or more chemical groups such as NH₂ or CH₃. In addition, the invention also covers amino acid sequences based on the amino acid sequences of SEQ ID NO:1 to SEQ ID NO:25 having a deletion, addition or substitution of one or more amino acids within said amino acid sequence.

The individual components within the pharmaceutical composition of the present invention can be present in varying quantities, which depends inter alia on the kind and form of formulation, the form of intended administration, the disease to be treated or the kind of vaccination. The pharmaceutical composition of the invention can be used, without being limited thereto, as solutions, suspensions, creams, ointments, powders for intraperitoneal administration, intranasal administration, subcutaneous administration, intradermal administration, intramuscular administration, peroral administration, intrarectal administration, intraocular administration, epidermal administration etc. In a particular preferred embodiment, the pharmaceutical composition of the present invention is adapted for topical application on the skin of a mammalian body, in particular on intact or lesional skin regions.

In a preferred embodiment of the present invention, the pharmaceutical composition comprises 0.0321 % to 4% by weight of the at least one oxidative stress substance such as dithranol, or other anthrones or hydroxyanthrecenes, 0.1% to 10% by weight of the at least one Toll-like receptor 7 (TLR7) ligand and 0.01% to 30% by weight of the at least one peptide antigen.

In a particularly preferred embodiment, the concentrations of the components of the pharmaceutical composition are as follows: 0.0625 % to 0.5% by weight of the at least one oxidative stress substance such as dithranol, or other anthrones or hydroxyanthrecenes, 3% to 5% by weight of the at least one Toll-like receptor 7 (TLR7) ligand and 0.1% to 0.4% by weight of the at least one peptide antigen.

The pharmaceutical composition may comprise a pharmaceutically acceptable vehicle, diluent, adjuvant or excipient which is adapted for the respective form of administration. As such, any diluent, adjuvant or excipient can be used, for example purified water, benzyl alcohol, cetyl alcohol, stearyl alcohol, polysorbate 80^{R}, sorbitan stearate, glycerol, methyl-4-hydroxy benzoate, propyl-4-hydrobenzoate, isostearic acid, xanthan gum. Also carriers, adjuvants or additives can be part of the pharmaceutical composition such as hydrophilic or lipophilic solvents, solubilizing agents, emulsifiers, viscosity-adjusting agents, pH-adjusting agents, chelating agents, preservatives, and/or agents for providing a basis for topical application or agent for increasing skin-penetrating capacity.

The pharmaceutical composition of the invention is in particular suitable to be used for a vaccination strategy against bacterial, viral, fungal infections, or against malign tumors, and is therefore preferably provided in the form of a vaccine. Particularly preferred is a transcutaneous immunization, in particular by means of topical administration. One advantage of a topical administration is the improvement of the compliance of patients, in particular patients suffering from a needle-phobia. As such the topical administration form is in particular useful for treatment of children or older patients. A further advantage is that a topical administration usually does not require medically qualified personnel in order to apply the medicament on the skin. The ability of the pharmaceutical composition or combination preparation to be administered in topical form allows a mass vaccination or immunization of both humans or animals, such as, for example, for the prevention or treatment of epidemic virus infections such as influenza. In veterinary medicine, economically destructive infection diseases such as swine influenza that occur in intensive animal husbandry are suitable targets for the pharmaceutical compositions or combination preparations of the present invention. Examples of animals to be treated are farm animals such as cattle, sheep and pigs, hog, goat chicken or gobbler. In addition, the inventive preparations may also be used for the treatment of black quarter (black leg caused by Clostridium chauvoei), foot and mouth disease (caused by Aphthoviruses), rabies (caused by lyssaviruses), blue tongue (caused by bluetongue virus), pox, brucellosis, listeriosis, campylobacter abortion, tuberculosis and paratuberculosis (Johne's disease), bovine ephemeral fever (caused by bovine ephemeral fever virus, BEFV), bovine rhinotracheitis (caused by bovine herpesvirus-1), PPR (goat plague, caused by morbilli viruses), babesiosis (caused by Babesia ssp), African swine fever and swine fever, avian flu, Schmallenberg virus.

Preferred cancer that can be treated by the present invention are: melamona and non-melanoma skin cancers, cancers of the gastrointenstinal tract (including colon, pancreas, liver) lung cancers, malignant lymphomas, acute and chronic leukemias and other cancers.

The synergistic effect of the three components of the inventive preparation allows specific immune responses against clearly defined peptide antigens in order to be able to treat persistent pathogen infections or tumor diseases. All three components contribute to said synergistic effect in relation to the induced immune response which was unexpected and surprising. The sole combination of a TLR7 ligand such as imiquimod together with a peptide antigen is not sufficient to induce a satisfactory sustained immune response suitable for therapeutic purposes. As shown in the present invention, the presence or co-administration of dithranol (or other anthrone or hydroxyanthracene compounds) leads to a synergy resulting in a much more sustained, prolonged immune response in comparison to an application of TLR7 ligand and peptide antigen alone. In particular, the three-component composition of the invention results in a strong stimulation of an adaptive immune response by stimulating cytotoxic T-cells and memory T-cells required for the induction of the desired therapeutic immune responses.

In a further aspect, the invention also relates to pharmaceutical combination preparations which contain the above mentioned at least one oxidative stress substance, at least one TLR7 ligand and at least one peptide antigen. Anything that has been described in regard of the pharmaceutical compositions also applies to the combination preparations and vice versa.

The oxidative stress substance can be either administered simultaneously or successively with the TLR7 ligand and/or peptide antigen, which depends on the kind of disease to be treated, the kind of vaccination and other parameters or factors affecting the formulation of a medicament or vaccine. In a preferred embodiment, the at least one oxidative stress substance is present in a separate formulation, wherein the at least one TLR7 ligand and/or the at least one peptide antigen are provided in one or more separate formulations. In a preferred embodiment, the at least one TLR7 ligand and the at least one peptide antigen are provided in a single formulation. In an alternative embodiment, the TLR7 ligand and the peptide antigen are provided in two separate formulations.

The present invention also relates to the use of such pharmaceutical combination preparations to be simultaneously or successively applied to a human or animal body. In this embodiment, the formulation of the at least one oxidative stress substance is adapted to be applied to a human or animal skin simultaneously or successively with the one or more formulations of the TLR7 ligand and/or peptide antigen. The treatment can be conducted as one-time treatment or as several successive treatments with identical or varying doses of the respective formulation.

In a preferred embodiment, the pharmaceutical combination preparation is adapted for topical administration on the skin of a patient. The topical administration of the oxidative stress substance such as dithranol induces an inflammatory milieu by means of oxidative mechanisms which allows antigen presenting cells (APC) to present the administered peptide antigen bearing an antigen epitope to the immune system, in particular to CD8+ T-cells. This mechanism is triggered by the at least one TLR7 ligand present in the formulation. The co-administration of the three components of the preparation surprisingly results in a strong primary cytotoxic T-cell response including a memory response already after one-time application. As illustrated by the present invention, the immune response has a long lasting effect due to the involvement of memory T-cells and is therefore extremely efficient in the elimination of pathogens such as bacteria, viruses or fungi from affected patients. The same strategy can also be applied for the treatment or prevention of malign tumors in which a target antigen epitope is known such that is can be presented as peptide antigen. Preferably, the peptide antigen of the invention bears a peptide epitope which is presented to APCs to elicit cytotoxic T-cell responses and memory T-cell responses.

The quantity of the individual components of the formulations of a pharmaceutical combination preparation can be selected by a person skilled in the art and can be adapted in accordance with the kind of treatment, the form and concentration of the ingredients and other parameters or factors that influence the therapeutic effect of the active agents. The treatment can be performed as a one-time treatment or be repeated several times using different or same quantities of the respective formulations.

The invention will be explained in more detail by the following examples. The examples are only for illustrative purposes and shall not limit the invention thereto.

### Examples:

Experiments were conducted in order to show the therapeutic and prophylactic effects of the pharmaceutical preparations of the present invention.

### Figure 1

Dithranol (Anthralin) enhances T cell responses after imiquimod based transcutaneous immunization (primary T cell response).

C57BL/6 mice (n= 4-12 per group) were immunized by TCI, performed as described by Lopez A. et al. (2017) using 50 mg IMI-Sol together with 100 µg of the synthetic peptides OVA ₂₅₇₋₂₆₄ (SIINFEKL; SEQ ID NO: 23) and OVA₂₂₃₋₂₃₇ (SIIQFEHL; SEQ ID NO: 24; obtained from peptides & elephants ,Potsdam, Germany) dissolved in DMSO and mixed with cremor basalis officinalis (Pamela Aranda Lopez et al., "Transcutaneous Immunization with a Novel Imiquimod Nanoemulsion Induces Superior T Cell Responses and Virus Protection.," Journal of Dermatological Science 87, no. 3 (September 2017): 252-59, doi:10.1016/j.jdermsci.2017.06.012). TCI treatments were applied via the ear skin on day 1.

Where indicated, dithranol (= anthralin at 0.0625% in Vaseline) was applied in the ear skin 24 h before. (A) The frequency of peptide-specific CD8⁺ T cells was assessed on day 7 via flow cytometry. (B) The *in vivo* cytolytic activity was analyzed on day 7, 20 h after transfer of peptide-loaded splenocytes. (C) IFN-γ production of splenocytes on day 7 post the indicated treatments. Splenocytes were re-stimulated with the indicated peptides for 24 h before harvest and analysis by ELISpot assay. (D) C57BL/6 mice (n= 5 per group) were immunized as in (A) and infected with OVA transgenic vaccinia virus (VV-OVA, 2x 10⁶ pfu *i.p.* 7 days later. 5 days after infection, mice were sacrificed and ovarial viral loads were determined by BSC-40 plaque assay.

*Significance by One Way ANOVA with Bonferroni Posttest analysis; p<0,05 (*).

### Conclusion:

The results demonstrate that dithranol treatment of the skin in combination with TCI has a synergistic effect on T cell activation on CD8 positive as well as CD4 positive T cells. Only the combined anthralin/TCI leads to a protective immune response against VV-OVA.

### Figure 2

Combined dithranol (anthralin) and imiquimod based transcutaneous immunization induces a memory immune response (secondary T cell response).

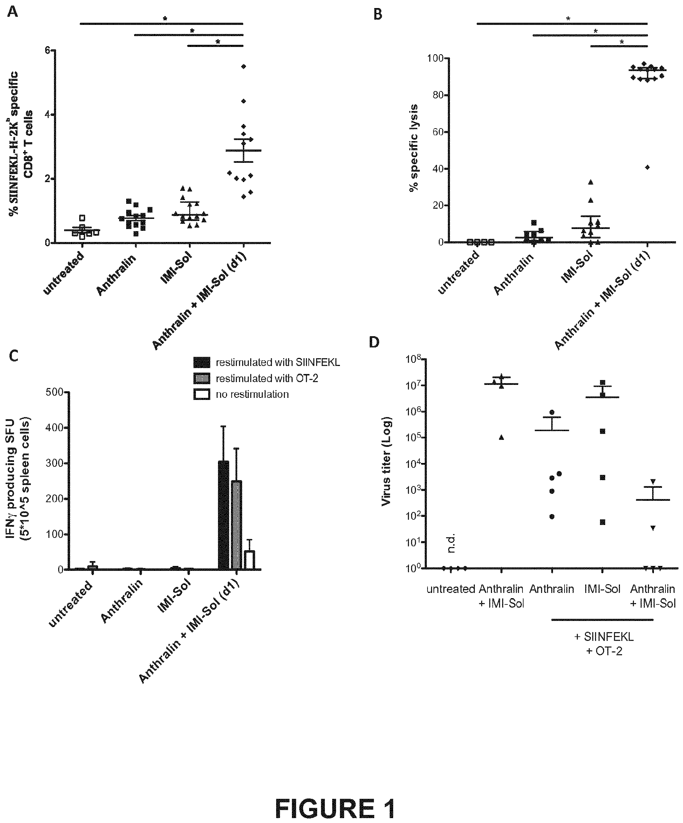

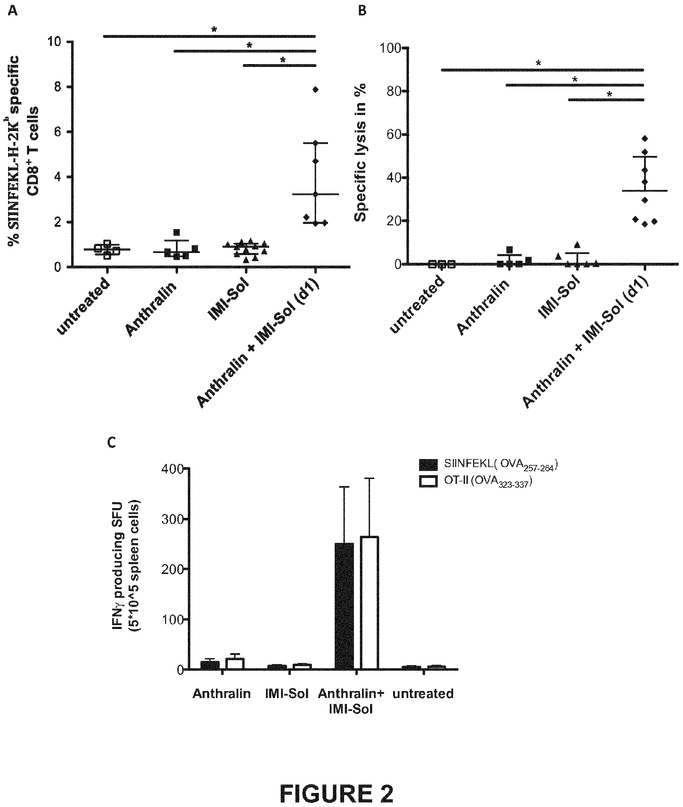

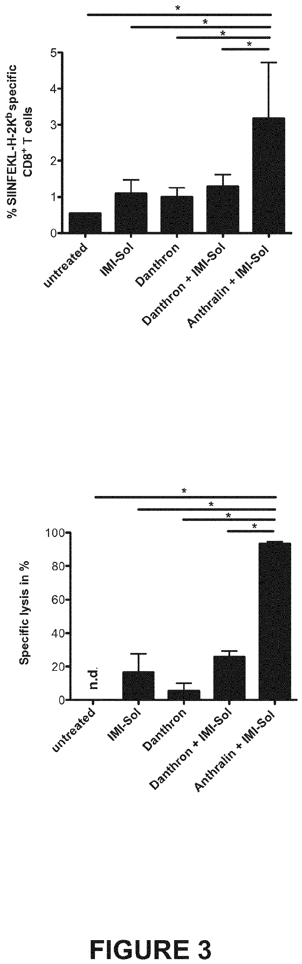

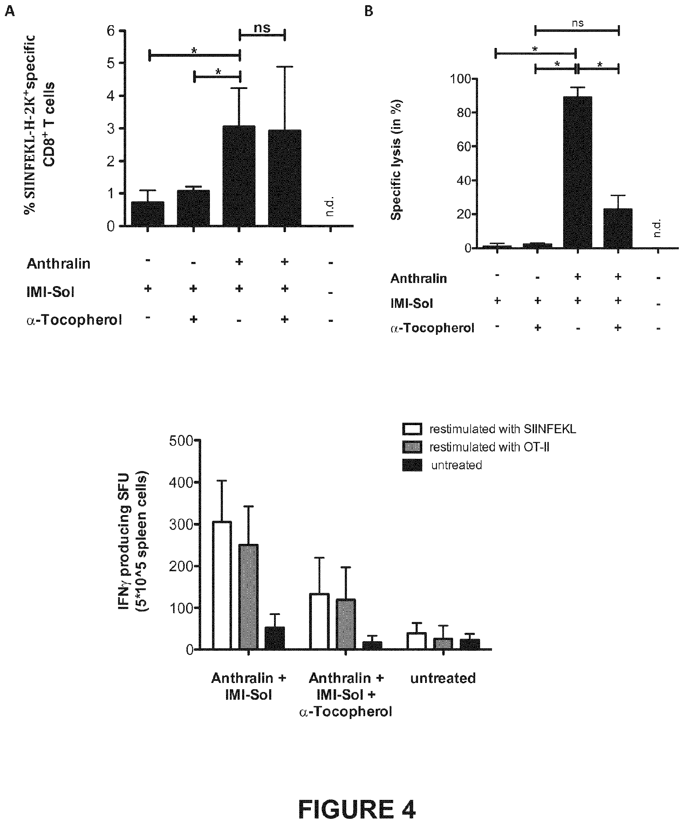

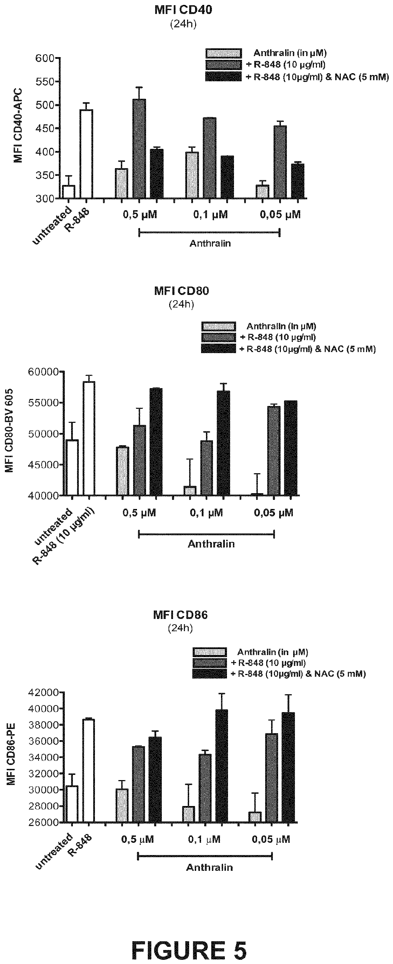

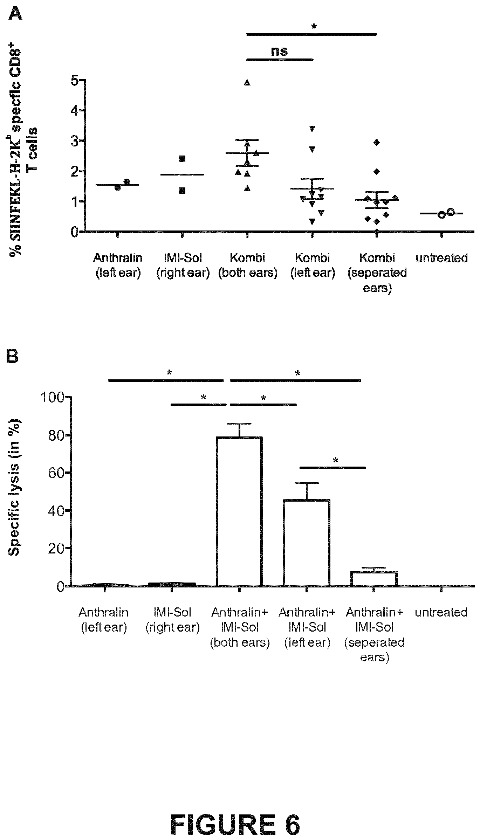

C57BL/6 mice (n= 3-8 per group) were immunized by TCI, performed as described in Fig. 1 (A) The frequency of peptide-specific CD8⁺ T cells was assessed on Day 35 via flow cytometry. (B) The *in vivo* cytolytic activity was analyzed on Day 35, 20 h after transfer of peptide-loaded splenocytes. (C) IFN-γ production of splenocytes on Day 35 post the indicated treatments. Splenocytes were re-stimulated with the indicated peptides for 24 h before harvest and analysis by ELISpot assay. *Significance by One Way ANOVA with Bonferroni Posttest analysis; p<0,05 (*).

### Conclusion:

The results demonstrate that dithranol treatment of the skin in combination with TCI has a synergistic effect on T cell activation on CD8 positive as well as CD4 positive T cells including memory T cell formation.

### Figure 3

Dithranol (anthralin), but not danthron enhances T cell responses after imiquimod based transcutaneous immunization (primary T cell response).

C57BL/6 mice (n=5 per group, untreated n = 2) were immunized by TCI, performed as described in Fig. 1. TCI treatments were applied via the ear skin on day 1. Where indicated Danthron (0.0625% in Vaseline) or dithranol (= anthralin at 0.0625% in Vaseline) applied in the ear skin 24 h before. (A) The frequency of peptide-specific CD8⁺ T cells was assessed on day 7 via flow cytometry. (B) The *in vivo* cytolytic activity was analyzed on day 7, 20 h after transfer of peptide-loaded splenocytes. *Significance by One Way ANOVA with Bonferroni Posttest analysis; p<0,05 (*).

### Conclusion:

The results demonstrate that dithranol treatment of the skin in combination with TCI has a synergistic effect on T cell activation. The oxidized analogue danthron has no impact on T cell activation suggesting that anthralin acts by oxidative mechanisms.

### Figure 4

α-Tocopherol antagonizes the enhanced cytolytic activity induced by dithranol (anthralin) TCI without affecting CTL frequency.

C57BL/6 mice (n= 4-10 per group) were immunized by TCI, performed as described in Fig. 1. TCI treatments were applied via the ear skin on day 1 either on both ears as indicated. Where indicated α-Tocopherol (600 U/ kg) as antioxidant was injected i.p. 1 h before the application of anthralin and/or TCI. (A) The frequency of peptide-specific CD8+ T cells was assessed on day 7 via flow cytometry. (B) The in vivo cytolytic activity was analyzed on day 7, 20 h after transfer of peptide-loaded splenocytes. (C) IFN-γ production of splenocytes on Day 7 post the indicated treatments.

*Significance by One Way ANOVA with Bonferroni Posttest analysis; p<0,05 (*).

### Conclusion:

The adjuvant effect of anthralin on TCI is dependent on oxidative mechanisms.

### Figure 5

Anthralin (dithranol) inhibits the TLR7 mediated activation of splenic dendritic cells *in vitro.*

CD11c positive Dendritic cells (DCs) were purified from spleens from C57BL/6 mice) as described in Weber M et al. (2014) and activated as indicated with the TLR7/8 agonist R-848 (10µg/ml), anthralin (dithranol), N-acetyl cysteine (NAC 5 mM) or left untreated in Iscoves's medium + 5% FCS (Michael Weber et al., "Donor and Host B Cell-Derived IL-10 Contributes to Suppression of Graft-Versus-Host Disease.," European Journal of Immunology 44, no. 6 (June 2014): 1857-65, doi:10.1002/eji.201344081).

After 24 h incubation, the cells were harvested, labeled with specific mAbs and analyzed by flow cytometry for the activation markers CD40, CD80 and CD86 gating on live (propidium iodide negative, CD1 1c/MHC class II positive DCs).

### Conclusion:

Anthralin suppresses the TLR7 mediated activation of DCs *in vitro.* The activation phenotype is restored by adding NAC as an antioxidant. Therefore, the synergistic effect of anthralin on T cell activation *in vivo* is surprising and unexpected. The restoration of DC activation in the presence of NAC indicates that anthralin acts by oxidative mechanisms

### Figure 6

Dithranol (anthralin) and TCI are required at the same location to enhance T cell responses (primary T cell response).

C57BL/6 mice (n= 2-9 per group) were immunized by TCI, performed as described in Fig. 1. TCI treatments were applied via the ear skin on day 1 either on both ears or only one ear as indicated. (A) The frequency of peptide-specific CD8⁺ T cells was assessed on day 7 via flow cytometry. (B) The *in vivo* cytolytic activity was analyzed on day 7, 20 h after transfer of peptide-loaded splenocytes.

*Significance by One Way ANOVA with Bonferroni Posttest analysis; p<0,05 (*).

### Conclusion:

Anthralin and TCI are required at the same site (ear) to mediate synergistic T cell activation. The magnitude of the induced T cell response upon Anthralin/TCI correlates with the area treated (dose-response relationship). Therefore, the synergistic effect of anthralin on T cell activation *in vivo* is initiated by the local activation of immune cells.

### Material & Methods

### Mice

6 to 8 weeks old C57BL/6 (wildtype) mice were used for vaccination experiments and purchased from the animal facility of the University of Mainz (TARC) or the Harlan Laboratories. All animal procedures were conducted according to the institutional guidelines and were reviewed and confirmed by an institutional review board headed by the local animal welfare officer (Prof. Dr. O. Kempski) of the University Medical Center (Mainz, Germany). All assays were approved by the responsible authority (National Investigation Office Rheinland-Pfalz, Koblenz, Germany). The Approval ID assigned by this authority: AZ 23 177-07/ G13-1-012.

### Transcutaneous Immunization

Transcutaneous immunizations were performed as described previously (Lopez et al. 2017) using 50 mg IMI-Sol (own production) together with 100 µg of the synthetic peptides OVA₂₅₇₋₂₆₄ (SIINFEKL; SEQ ID NO: 23) and OVA₂₂₃₋₂₃₇ (SIIQFEHL; SEQ ID NO: 24; obtained from peptides & elephants Potsdam, Germany) dissolved in DMSO and mixed with cremor basalis officinalis. TCI treatments were applied via the ear skin on day 1.

Foregoing to TCI, ears were pretreated with 25 mg of an anthralin or danthron containing (1/16 %) vaseline creme on day 0 when indicated. α-Tocopherol was administered in a weight adjusted dosage (600 U/ kg body weight, i.p.) 1 h preceding the application of anthralin and/or TCI. Mice were anesthetized previous to all procedures using a dilution of Ketamin/Rompun in a weight adjusted dosage.

### Flow cytometric analyses and in vivo cytotoxicity assay

Flow cytometric analyses and evaluation of *in vivo* cytotoxicity were performed as described previously (Lopez et al. 2017). Blood samples were collected via tail vein incision, subjected to hypotonic lysis and incubated with specific mAbs as indicated. Antibodies used for FACS analysis were PB-conjugated anti-CD8 (clone 53-6.7; eBioscience, San Diego, USA), FITC-conjugated anti-CD62L (clone 17A2; eBioscience, San Diego, USA) and APC-conjugated anti-CD44 (clone GK1.5; BioLegend, San Diego, USA). To measure the frequency of peptide-specific CD8⁺ T cells, blood samples were stained with PE-conjugated OVA₂₅₇₋₂₆₄-H2-K^{b} (tetrameric labeling, own production).

For detecting *in vivo* cytolytic activity, splenocytes of syngenic wildtype mice were labeled with different amounts of 5,6-carboxyfluorescein diacetate succinimidyl ester (CFSE; Invitrogen, Carlsbad, USA) resulting in a CFSE^{low} and a CFSE^{high} population. The cells labeled with CFSE^{low} were additionally labeled with 1µM of OVA₂₅₇₋₂₆₄. Adoptive transfer of 3x10⁷ cells in a 1:1 ratio (CFSE^{low}:CFSE^{high}) into immunized and untreated mice was performed via intravenous injection into the tail vein. For assessment of cytolytic activity during the primary response, blood was drawn and subjected to flow cytometry on day 7 post immunization, 20 hours after transfer of target cells. Specific lysis was evaluated as following: Specific lysis (%) = 100x (1- (CFSE^{low} of immunized mouse/CFSE^{low} of untreated control). All analyses were performed with a LSRII Flow Cytometer and FACSDiva Software (Becton Dickinson, Franklin Lakes, USA).

### IFNv Elispot assay

96 well plates (MultiScreen_{HTS} IP, 0.45mm, Merck Millipore, Darmstadt, Germany) were coated over night at 4 °C with murine IFNγ AN18 antibody (10 mg/ml, Mabtech, Nacka Strand, Sweden). After a blocking step 5x10⁵ cells were added. Therefore splenocytes were lysed and loaded either with 1mM OVA₂₅₇₋₂₆₄, 1 mM OVA₃₂₃₋₃₃₇ or left in medium. After over night incubation at 37 °C plates were washed and stained with the biotinylated IFNγ R4-6A2 antibody (2 mg/ml, Mabtech, 2 h, 37 °C). Afterwards Vectastain ABC Kit (Vector Laboratories, Burlingame, USA)/AEC (Sigma-Aldrich, Taufkirchen, Germany) complex was added as described in the manufacturer's instruction. After spots were visible plates were washed with water and dried overnight. Analysis was performed by an ImmunoSpot Analyzer (C.T.L. Europe, Bonn, Germany).

### Dendritic cell isolation, cultivation and assessment of activation markers

Spleens of C57BL6/J wildtype mice were harvested, picked and digested for 45 min with type 2 collagenase (50U/ml from Worcester, Pappenheim, Germany) and stopped by adding 2 mM EDTA (from Sigma-Aldrich, Taufkirchen, Germany). After a hypotonic lysis step DCs were isolated using CD11c MicroBeads UltraPure (Miltenyi Biotec, Germany) following the manufacturer's instruction. 2x10⁵CD11c⁺ DCs/ well were plated in 96 well plates and cultivated for either 24 or 48 h in the presence of TLR7 ligand R-848 (10 ug/ml) and/or different concentrations of anthralin (solved in DMSO). Afterwards cells were washed and stained with following antibodies for FACS analysis: CD19-PerCP, CD90.2-PerCP, CD11c-APC or PE-Cy7, MHCII-BV241, CD86-PE, CD80-FITC or BV605, CD40-APC. Additionally, supernatants of DC cultivation after 24 or 48 h were collected and employed for cytokine bead array (CBA) analyses. Following cytokines were quantified using CBA technology: IL-1b; IL-6; IL-10; IL-12; IL-23; TNF-α.

### Assessment of virus protection via plaques assay

Prior to the infection female C57BL6/J mice were immunized as described above. On day 7 effective immunization was verified via tetrameric staining. Afterwards 2x10⁶ pfu Vaccinia OVA virus (VV OVA) were re-suspended in 200 µl medium and applied via intraperitoneal injection. 5 days after the infection mice were sacrificed and ovaries were removed and minced. Different concentrations of these virus containing samples were added to a layer of BSC-40 cells. Subsequent the plates were incubated for 24 h at 37 °C. Following this incubation an examination under the light microscope revealed the appearance of plaques within the cell layer. Consecutively, the remaining BSC-40 cells were stained with a crystal violet solution and plaques were counted on a lightbox. Counted plaques were multiplicated with the dilution factor of the appropriate well to estimate the pfu/ovary.

### Tumor rejection assay

MC38 colon adenocarcinoma cells were provided by H.C. Probst (Institute for Immunology, University Medical Center Mainz). Before inoculation, MC38 cells were cultured for one week in DMEM (Thermo Fisher Scientific), supplemented with 1% penicillin-streptomycin, 10% FCS, 2mM glutamine and 1mM sodium pyruvate. Tumor cells were subcutaneously injected into the lower left flank of anesthetized animals. Treatment was initiated at tumor size of approximately 25 mm² (5-7 days after inoculation). Tumor growth was traced by measuring the tumor diameter in 2 dimensions three times a week using a caliper. Mice were sacrificed when tumor size exceeded a diameter of 400 mm² or when bleeding ulceration occurred. The point of death was recorded as the day after sacrifice.

## Claims

1. A pharmaceutical composition, comprising at least the following components:
(a) at least one oxidative stress substance selected from the group consisting of dithranol (anthralin, cignolin), or other anthrones or hydroxyanthracenes,
(b) at least one Toll-like receptor 7 (TLR7) ligand,
(c) at least one peptide antigen.

2. The pharmaceutical composition according to claim 1, wherein the at least one TLR7 ligand is imiquimod (R837), loxoribine and/or resiquimod (R848).

3. The pharmaceutical composition according to claim 1, wherein the at least one TLR7 ligand is Pam3Cys, poly-(I:C) or CpG-DNA

4. The pharmaceutical composition according to anyone of claims 1 to 3, wherein the at least one peptide antigen is selected from the group consisting of (i) major histocompatibility complex class I (MHCI) ligands, (ii) major histocompatibility complex class II (MHCII) ligands, or (iii) peptides that comprise one or more of said MHCI and/or MHCII ligands.

5. The pharmaceutical composition according to claim 1, wherein the at least one peptide antigen is a synthetic peptide or isolated naturally occurring peptide comprising the amino acid sequence of SEQ ID NO: 1 (PLDGEYFTL), SEQ ID NO: 2 (YMNGTMSQV),SEQ ID NO: 3 (AAGIGILTV), SEQ ID NO: 4 (VLRENTSPK), SEQ ID NO: 5 (SPSSNRIRNT), SEQ ID NO: 6 (RLVTLKDIV), SEQ ID NO: 7 (AVFDRKSDAK), SEQ ID NO: 8 (GLSPTVWLSV), SEQ ID NO: 9 (ILKEPVHGV), SEQ ID NO: 10 (KIRLRPGGK), SEQ ID NO: 11 (TPGPGVRYPL), SEQ ID NO: 12 (ILGFVFTLTV), SEQ ID NO: 13 (VLTDGNPPEV), SEQ ID NO: 14 (TQHFVQENYLEY), SEQ ID NO: 15 (WRRAPAPGAKAMAPG), SEQ ID NO: 16 (FRKQNPDIVIQYMDDLYVG), SEQ ID NO: 17 (RIHIGPGRAFYTTKNIIGTI), SEQ ID NO: 18 (PGPLRESIVCYFMVFLQTHI), SEQ ID NO: 19 (PYYTGEHAKAIGN), ), SEQ ID NO: 20, (IAFNSGMEPGVVAEKV), SEQ ID NO: 21 (KQEELERDLRKTKKKI), SEQ ID NO: 22 (GRDIKVQFQSGGNNSPAV), SEQ ID NO: 23 (SIINFEKL), SEQ ID NO: 24 (SIIQFEHL), SEQ ID NO: 25 (SGPSNTPPEI) and modified forms thereof in which one or more amino acids in said amino acid sequences are linked to a chemical group, preferably NH₂ or CH₃.

6. The pharmaceutical composition according to anyone of claims 1 to 5, wherein the composition comprises 0.3125 % to 4 % by weight of the at least one oxidative stress substance, 0.1% to 10% by weight of the at least one Toll-like receptor 7 (TLR7) ligand and 0.01% to 30% by weight of the at least one peptide antigen.

7. The pharmaceutical composition according to anyone of claims 1 to 6, wherein the three components of the composition are present in a form which is adapted for topical administration on intact or lesional skin regions.

8. The pharmaceutical composition according to anyone of claims 1 to 7, wherein the composition further comprises a pharmaceutically acceptable vehicle, diluent, adjuvant or excipient.

9. The pharmaceutical composition according to anyone of claims 1 to 8, wherein the composition is provided in form of a vaccine.

10. A pharmaceutical composition according to anyone of claim 1 to 9 for use in the prevention or treatment of a persistent viral, bacterial or fungal infection.

11. A pharmaceutical composition according to anyone of claim 1 to 9 for use in the prevention or treatment of cancer.

12. A pharmaceutical combination preparation, comprising at least the following components:
(a) at least one oxidative stress substance selected from the group consisting of dithranol (anthralin, cignolin), or other anthrones or hydroxyanthracenes,
(b) at least one Toll-like receptor 7 (TLR7) ligand,
(c) at least one peptide antigen,
wherein component (a) and components (b) and (c) are present in at least two separate formulations.

13. The pharmaceutical combination preparation according to claim 11, wherein the components (a), (b) and (c) in the formulations are defined as in anyone of claims 1 to 9.

14. The pharmaceutical combination preparation according to claim 12 or claim 13, wherein the formulation of component (a) is adapted to be applied to a human or animal body simultaneously or successively with the formulations of components (b) and (c).

15. A pharmaceutical combination preparation according to anyone of claim 12 to 14 for use in the prevention or treatment of a persistent viral, bacterial or fungal infection.

16. A pharmaceutical combination preparation according to anyone of claim 12 to 14 for use in the prevention or treatment of cancer.


SEQUENCE LISTING

<110>  Universitätsmedizin der Johannes Gutenberg-Universität
       Mainz

<120>  Pharmaceutical compositions, vaccines and their uses in the
       prevention or treatment of a persistent infection or of cancer

<130>  Dreifachimpfstoff

<160>  25

<170>  PatentIn version 3.5

<210>  1
<211>  9
<212>  PRT
<213>  Unknown

<220>
<223>  Mammal P53 322-330

<400>  1

Pro Leu Asp Gly Glu Tyr Phe Thr Leu
1               5


<210>  2
<211>  9
<212>  PRT
<213>  Unknown

<220>
<223>  Tyrosinase 351-359

<400>  2

Tyr Met Asn Gly Thr Met Ser Gln Val
1               5


<210>  3
<211>  9
<212>  PRT
<213>  Unknown

<220>
<223>  Melan A/Mart-1 27-35

<400>  3

Ala Ala Gly Ile Gly Ile Leu Thr Val
1               5


<210>  4
<211>  9
<212>  PRT
<213>  Unknown

<220>
<223>  HER-2 754-762

<400>  4

Val Leu Arg Glu Asn Thr Ser Pro Lys
1               5


<210>  5
<211>  10
<212>  PRT
<213>  Unknown

<220>
<223>  RAGE-1 (Orf2) 11-20

<400>  5

Ser Pro Ser Ser Asn Arg Ile Arg Asn Thr
1               5                   10


<210>  6
<211>  9
<212>  PRT
<213>  Human papillomavirus type 11

<400>  6

Arg Leu Val Thr Leu Lys Asp Ile Val
1               5


<210>  7
<211>  10
<212>  PRT
<213>  Epstein-Barr virus

<400>  7

Ala Val Phe Asp Arg Lys Ser Asp Ala Lys
1               5                   10


<210>  8
<211>  10
<212>  PRT
<213>  Hepatitis B virus

<400>  8

Gly Leu Ser Pro Thr Val Trp Leu Ser Val
1               5                   10


<210>  9
<211>  9
<212>  PRT
<213>  Human immunodeficiency virus type 1

<400>  9

Ile Leu Lys Glu Pro Val His Gly Val
1               5

<210>  10
<211>  9
<212>  PRT
<213>  Human immunodeficiency virus type 1

<400>  10

Lys Ile Arg Leu Arg Pro Gly Gly Lys
1               5


<210>  11
<211>  10
<212>  PRT
<213>  Human immunodeficiency virus type 1

<400>  11

Thr Pro Gly Pro Gly Val Arg Tyr Pro Leu
1               5                   10


<210>  12
<211>  10
<212>  PRT
<213>  Influenza A virus

<400>  12

Ile Leu Gly Phe Val Phe Thr Leu Thr Val
1               5                   10


<210>  13
<211>  10
<212>  PRT
<213>  Mycobacterium tuberculosis

<400>  13

Val Leu Thr Asp Gly Asn Pro Pro Glu Val
1               5                   10


<210>  14
<211>  12
<212>  PRT
<213>  Unknown

<220>
<223>  MAGEA3 247-258

<400>  14

Thr Gln His Phe Val Gln Glu Asn Tyr Leu Glu Tyr
1               5                   10


<210>  15
<211>  15
<212>  PRT

<213>  Unknown

<220>
<223>  LDL receptor FUT fusion protein 315-330

<400>  15

Trp Arg Arg Ala Pro Ala Pro Gly Ala Lys Ala Met Ala Pro Gly
1               5                   10                  15


<210>  16
<211>  19
<212>  PRT
<213>  Human immunodeficiency virus type 1

<400>  16

Phe Arg Lys Gln Asn Pro Asp Ile Val Ile Gln Tyr Met Asp Asp Leu
1               5                   10                  15


Tyr Val Gly



<210>  17
<211>  20
<212>  PRT
<213>  Human immunodeficiency virus type 1

<400>  17

Arg Ile His Ile Gly Pro Gly Arg Ala Phe Tyr Thr Thr Lys Asn Ile
1               5                   10                  15


Ile Gly Thr Ile
            20


<210>  18
<211>  20
<212>  PRT
<213>  Epstein-Barr virus

<400>  18

Pro Gly Pro Leu Arg Glu Ser Ile Val Cys Tyr Phe Met Val Phe Leu
1               5                   10                  15


Gln Thr His Ile
            20


<210>  19
<211>  13
<212>  PRT
<213>  Influenza B virus

<400>  19

Pro Tyr Tyr Thr Gly Glu His Ala Lys Ala Ile Gly Asn
1               5                   10

<210>  20
<211>  16
<212>  PRT
<213>  Mycobacterium leprae

<400>  20

Ile Ala Phe Asn Ser Gly Met Glu Pro Gly Val Val Ala Glu Lys Val
1               5                   10                  15

<210>  21
<211>  16
<212>  PRT
<213>  Hepatitis delta virus

<400>  21

Lys Gln Glu Glu Leu Glu Arg Asp Leu Arg Lys Thr Lys Lys Lys Ile
1               5                   10                  15

<210>  22
<211>  18
<212>  PRT
<213>  Mycobacterium tuberculosis

<400>  22

Gly Arg Asp Ile Lys Val Gln Phe Gln Ser Gly Gly Asn Asn Ser Pro
1               5                   10                  15

Ala Val

<210>  23
<211>  8
<212>  PRT
<213>  Unknown

<220>
<223>  Ovarium albumin 257-264

<400>  23

Ser Ile Ile Asn Phe Glu Lys Leu
1               5

<210>  24
<211>  8
<212>  PRT
<213>  Unknown

<220>
<223>  Ovarium albumin 323-337

<400>  24

Ser Ile Ile Gln Phe Glu His Leu
1               5


<210>  25
<211>  10
<212>  PRT
<213>  Adenovirus type 5

<400>  25

Ser Gly Pro Ser Asn Thr Pro Pro Glu Ile
1               5                   10